# EUROPÄISCHE PATENTANMELDUNG

(11) **EP 2 799 010 A1**
(43) Veröffentlichungstag der Anmeldung: **05.11.2014**
(21) Anmeldenummer: 14001430.9
(22) Anmeldetag: 19.04.2014
(51) Int. Cl.: A61B 5/145, A61B 5/00, A61B 5/1455

(54) **Verfahren und Vorrichtung zur nicht-invasiven Bestimmung einer Messgröße eines Analyten in einem biologischen Körper**

(30) Priorität: 04.05.2013 DE 102013007776; 16.05.2013 DE 102013008400
(71) Anmelder: SAMTD GmbH & Co. KG, 90478 Nürnberg (DE)
(72) Erfinder: Glasmacher, Mathias, DE - 68799 Reilingen (DE); Tholl, Hans Dieter, DE - 88682 Salem (DE)
(74) Vertreter: Diehl Patentabteilung

(57) **Zusammenfassung**

Es werden ein Verfahren und eine Vorrichtung (1, 2) zur nicht-invasiven Bestimmung einer Messgröße eines Analyten in einem biologischen Körper (4) angegeben, wobei automatisiert von einem Körper (4) ausgehendes Licht spektralaufgelöst detektiert und ein Spektrum des detektierten Lichts gewonnen wird, und wobei in dem Spektrum ein für den Analyten charakteristischer Verlauf identifiziert und aus dem identifizierten Verlauf ein Wert für die Messgröße ermittelt wird. Weiter wird automatisiert am Körper (4) unter Variation des Beobachtungsortes (17, 18) eine Mehrzahl von Spektren gewonnen und hieraus eine Mehrzahl von jeweils eine Ortsinformation beinhaltenden Werten der Messgröße ermittelt. Durch einen Vergleich der ermittelten Werte der Messgröße wird eine Strukturinformation des Körpers (4) abgeleitet und aus der Strukturinformation auf den Wert der Messgröße in einer bestimmten Struktur des Körpers (4) geschlossen. Dieser Wert der Messgröße wird ausgegeben.

## Beschreibung

Die Erfindung betrifft ein Verfahren zur nicht-invasiven Bestimmung einer Messgröße eines Analyten in einem biologischen Körper, wobei automatisiert vom beleuchteten Körper ausgehendes Licht unterschiedlicher Wellenlängen detektiert und ein Spektrum des detektierten Lichts gewonnen wird, und wobei in dem Spektrum ein für den Analyten charakteristischer Verlauf identifiziert und aus dem identifizierten Verlauf ein Wert für die Messgröße ermittelt wird. Die Erfindung betrifft weiter eine zur Durchführung des Verfahrens entsprechend ausgebildete Vorrichtung. Insbesondere beschäftigt sich die Erfindung mit dem Problem einer nicht-invasiven Bestimmung des Blutzuckerspiegels.

Grundsätzlich ist es wünschenswert, biomedizinische Parameter in-vivo und nicht-invasiv bestimmen zu können. Damit könnten insbesondere für eine Diagnose erforderliche und wiederkehrend oder häufig zu überprüfende Parameter schmerzfrei und ohne einen Eingriff an einem menschlichen oder tierischen Körper ermittelt werden. Gerade eine nicht-invasive Bestimmung einer Messgröße eines im Körper enthaltenen Analyten gestaltet sich jedoch schwierig, da durch die Vielzahl von sich in ihren physikalischen und chemischen Parametern unterscheidenden Strukturen eines Körpers, wie Haut, Muskeln, Sehnen, Knochen, Gefäße, Fettgewebe und Organgewebe, der Erhalt von konkret einem Analyten zuordenbaren Messsignalen erschwert ist. Zudem sind Messsignale aus dem Körperinneren grundsätzlich mit einem vergleichsweise schlechten Signal-zu-Rausch-Verhältnis belegt. Muss die Messgröße des Analyten für eine konkrete Struktur, beispielsweise in einem Blutgefäß, ermittelt werden, so wird das Messergebnis zusätzlich durch die Umgebung verfälscht, wenn dort die Messgröße des Analyten einen anderen Wert aufweist. Interessierende Analyten sind beispielsweise Zucker, insbesondere Glucose, Alkohol, Drogen, Fette und Wasser, aber auch Hormone, Botenstoffe, Enzyme, Spurenelemente, Mineralien, Metalle, Medikamente und toxische Substanzen. Die Analyten können hierbei in fester, gasförmiger oder flüssiger Form vorliegen, wobei der Analyt insbesondere als eine Lösung in Körperflüssigkeiten oder in Körpergewebe gegeben sein kann.

Zu einer nicht-invasiven Ermittlung von biomedizinischen Parametern eignen sich insbesondere optische Methoden, die durch Streuung, Transmission, Absorption, Reflexion, Polarisation, Phasenänderung, Fluoreszenz, photoakustische Anregung oder photothermische Anregung in der Lage sind, die Anwesenheit des gesuchten Analyten zu detektieren. Je nach Methode kann aus dem Detektionssignal dann mit einer entsprechenden Messgenauigkeit ein Wert für die gewünschte Messgröße, wie beispielsweise ein Wert für eine Konzentration, bestimmt werden.

Aus M. A. Pleitez et al., "In Vivo Noninvasive Monitoring of Glucose Concentration in Human Epidermis by Mid-Infrared Pulsed Photoacoustic Spectroscopy", Analytical Chemistry 2013, Bd. 85 (2), S. 1013-1020 ist beispielsweise eine photoakustische Methode zur Bestimmung der Konzentration von Glucose in menschlicher Epidermis bekannt. Aus Z. Zalevsky, J. Garcia, "Laserbasierte biomedizinische Untersuchungen - simultan und kontaktlos", BioPhotonic 3, 2012, S. 30 - 33 ist weiter eine nicht-invasive Methode zur Bestimmung des Alkohol- und des Glucosespiegels in Blut bekannt, wobei durch Beobachtung von Speckle-Mustern Hautvibrationen vermessen werden. Dazu wird die Haut mit einem Laser diffus beleuchtet und die durch Interferometrie gebildeten Speckle-Muster nachverfolgt. Ferner ist aus K.-U. Jagemann et al., "Application of Near-Infrared Spectroscopy for Non-Invasive Determination of Blood/Tissue Glucose Using Neural Networks", Zeitschrift für Physikalische Chemie, Bd. 191, 1995, S. 179 - 190 eine nicht-invasive Bestimmung von Glucose in Blut oder Gewebe mittels NIR-Spektroskopie bekannt.

Die bisher bekannten Methoden zu einer in-vivo nicht-invasiven Bestimmung einer Messgröße eines Analyten in einem biologischen Körper erreichen zum Teil nicht die für klinische Anwendungen gewünschten Messgenauigkeiten. Einige dieser Methoden sind zudem aufgrund der Komplexität der erforderlichen Messapparaturen und aufgrund der für die Messung benötigten Zeit nicht geeignet, von Patienten zu einer eigenen regelmäßigen Kontrolle der entsprechenden Messgröße des Analyten herangezogen bzw. benutzt zu werden. Insbesondere gibt es zur Bestimmung der Blutzuckerkonzentration noch kein nicht-invasives Verfahren, welches von an Diabetes erkrankten Personen selbst zu einer regelmäßigen Kontrolle zuhause eingesetzt werden könnte. Gleichwohl wäre dies wünschenswert, da die bisherigen Verfahren regelmäßig eine gegebenenfalls schmerzhafte Blutentnahme erforderlich machen bzw. eine in gewissen Situationen sinnhafte, in kurzen Zeitabständen wiederholte Messung nicht praktikabel ist.

Der Erfindung liegt daher die Aufgabe zugrunde, ein alternatives nicht-invasives Verfahren zur Bestimmung einer Messgröße eines Analyten in einem biologischen Körper anzugeben, welches das Potential für eine Anwendung im Consumer-Markt oder im klinischen Alltag bietet, so dass der Patient eigenständig Messungen zur Kontrolle der jeweiligen Messgröße durchführen kann.

Weiter liegt der Erfindung die Aufgabe zugrunde, eine zur Durchführung des angegebenen Verfahrens geeignete Vorrichtung anzugeben, die die Möglichkeit zu einer Weiterentwicklung in ein Consumer-Produkt oder für den klinischen Alltag bietet.

Die erstgenannte Aufgabe wird erfindungsgemäß durch ein Verfahren zur nicht-invasiven Bestimmung einer Messgröße eines Analyten in einem biologischen Körper gelöst, wobei automatisiert vom einem Körper ausgehendes Licht detektiert und ein Spektrum des detektierten Lichts gewonnen wird, und wobei in dem Spektrum ein für den Analyten charakteristischer Verlauf identifiziert und aus dem identifizierten Verlauf ein Wert für die Messgröße ermittelt wird. Dabei ist weiter vorgesehen, dass - jeweils automatisiert - am Körper unter Variation des Beobachtungsortes eine Mehrzahl von Spektren gewonnen und hieraus eine Mehrzahl von jeweils eine Ortsinformation beinhaltenden Werten der Messgröße ermittelt wird, dass durch einen Vergleich der ermittelten Werte der Messgröße eine Strukturinformation des Körpers abgeleitet und aus der Strukturinformation auf den Wert der Messgröße in einer bestimmten Struktur des Körpers geschlossen wird, und dass dieser Wert der Messgröße ausgegeben wird. Der automatisiert ermittelte und ausgegebene Wert kann dann die Grundlage für eine nachfolgende Diagnose sein.

Die Erfindung geht dabei in einem ersten Schritt von der Überlegung aus, dass es aufgrund der in einem biologischen Körper vorhandenen, sich in ihren physikalischen und chemischen Parametern unterscheidenden Strukturen nicht unmittelbar möglich ist, aus einem erhaltenen optischen Messsignal auf einen konkreten Wert der betrachteten Messgröße des Analyten zu schließen. Insbesondere ist dies der Fall, wenn sich die Werte der Messgröße in den verschiedenen Strukturen des Körpers unterscheiden. Beispielsweise wird sich die Konzentration eines fettlöslichen oder wasserlöslichen Analyten im Fettgewebe von der Konzentration in einer Körperflüssigkeit, wie z. B. in Blut oder in Lymphe, unterscheiden.

In einem zweiten Schritt macht sich die Erfindung überraschend gerade die vorgenannte Überlegung zunutze, indem sie erkennt, dass aus den an verschiedenen Beobachtungsorten gewonnenen Werten für die betrachtete Messgröße des Analyten eine Strukturinformation des Körpers abgeleitet werden kann.

Wird beispielsweise der Beobachtungsort für den Erhalt des gemessenen Spektrums durch verschiedene Strukturen des Körpers, beispielsweise durch Haut, Fettgewebe, Gefäße und Knochen, bewegt, so resultiert hieraus aufgrund der unterschiedlichen Anreicherung des Analyten ein der Variation des Beobachtungsortes entsprechender Verlauf der jeweils ermittelten Werte der Messgröße. Mit anderen Worten kann aus dem ortsabhängig ermittelten Verlauf der Werte der betrachteten Messgröße eine Strukturinformation für den Körper bzw. für das untersuchte Köpervolumen abgeleitet werden. Daher wird es unter Zugrundelegung der an sich bekannten Anatomie des Körpers möglich, aus dem ermittelten Verlauf der Werte für die Messgröße des Analyten darauf zu schließen, aus welcher Struktur des Körpers ein jeweils spezifisch ermittelter Wert stammt. Dieser konkrete Wert kann dann als gesuchter Wert der Messgröße des Analyten in der gesuchten Struktur ausgegeben werden. Insbesondere wird es möglich, den Wert der betrachteten Messgröße des Analyten beispielsweise für eine Körperflüssigkeit wie Blut oder Lymphe in einem jeweiligen Gefäß anzugeben, welches in Haut- oder Fettgewebe etc. eingebettet ist. Aus dem durch Ortsvariation ermittelten Verlauf der Werte für die betrachtete Messgröße des Analyten wird also auf eine konkret gesuchte Struktur geschlossen, und für diese Struktur der zugeordnete Wert der Messgröße ausgegeben.

Zur Ermittlung des Wertes der Messgröße wird ein optisches Verfahren eingesetzt. Insbesondere wird ein spektroskopisches Verfahren verwendet, da sich hierin charakteristische Signaturen des Analyten vergleichsweise leicht feststellen lassen. Als ein charakteristischer Verlauf wird in dem Spektrum insofern bevorzugt wenigstens ein Abschnitt einer spezifischen Spektralsignatur des Analyten identifiziert. Durch Mittelung über einen bestimmten Spektralbereich, durch Betrachtung der Spitzenintensitäten oder durch spezifische mathematische Auswerte-, Rauschunterdrückungs- und/oder Glättungsverfahren lässt sich gegebenenfalls unter zusätzlicher Addierung über mehrere Spektren ein konkreter Wert der Messgröße des jeweils untersuchten Analyten ermitteln. In aller Regel lässt sich bei spektroskopischen Verfahren auch bei niedrigen Signalintensitäten die Signatur des untersuchten Analyten noch vom Untergrundrauschen oder von Signaturen anderer Bestandteile trennen.

Zur Durchführung des angegebenen Verfahrens eignet sich grundsätzlich jede spektroskopische Methode, die es ermöglicht, in einem vom Körper ausgehenden Licht eine spezifische Signatur des Analyten zu identifizieren. Dabei ist es möglich, das Spektrum in der vom Körper ausgehenden, aus bestimmten Tiefenregionen emittierten intrinsischen Wärmestrahlung oder im Falle des Einsatzes einer externen Lichtquelle in einer Transmissions- oder in einer Reflexionsrichtung zur Beleuchtung zu beobachten. Auch kann in einem Streusignal eine Signatur des Analyten identifiziert werden. Weiter kann ein Fluorenzenzspektrum oder ein sonstiges Emissionsspektrum des Analyten beobachtet werden. Die Detektion des vom Körper ausgehenden Lichts kann sowohl breitbandig als auch spektral aufgelöst erfolgen.

In einer vorteilhaften Ausgestaltung wird der Körper mit Licht unterschiedlicher Wellenlängen beleuchtet, wobei das vom beleuchteten Körper ausgehende Licht detektiert wird. Diese Variante bietet den Vorteil, dass über die Intensität der Lichtquelle das Signal-zu-Rausch-Verhältnis im detektierten Licht verbessert werden kann. Zudem können über die Lichtquelle spezifische, für die Signatur des Analyten bevorzugte Wellenlängen bzw. Wellenlängenbereiche eingestellt werden.

Aufgrund der Unterscheidbarkeit von medizinisch interessanten Analyten in ihren IR-Schwingungsspektren wird der Körper in einer bevorzugten Variante mit Licht im IR-Spektralbereich beleuchtet. In einer Variante wird der Körper mit einer durchstimmbaren Lichtquelle beleuchtet. Insbesondere wird eine Lichtquelle eingesetzt, die in einem IR-Teilbereich durchstimmbar ist, der auf charakteristische IR-Schwingungsbanden des untersuchten Analyten abgestimmt ist. Als eine hierfür geeignete Quelle wird bevorzugt ein Quantenkaskadenlaser eingesetzt. Ein derartiger Laser emittiert mit hoher Güte mit einer Wellenlänge innerhalb des IR-Spektralbereichs und weist eine Durchstimmbarkeit von etwa 20 % der Zentralwellenlänge auf. Beispielsweise kann ein Quantenkaskadenlaser eingesetzt sein, der durchstimmbar Licht mit einer Wellenlänge zwischen 8,5 µm und 10,5 µm emittiert. In diesem Bereich liegen charakteristische Absorptionsbanden von Glucose, die sich dementsprechend auch in einem Reflexionsspektrum beobachten lassen.

In einer anderen Variante wird zur Beleuchtung des Körpers eine breitbandig emittierende Lichtquelle eingesetzt werden. Als eine im IR-Bereich oder in einem IR-Teilbereich emittierende Lichtquelle kann insbesondere eine Wärmequelle eingesetzt sein. Im Unterschied zu einer durchstimmbaren Lichtquelle erlaubt eine breitbandig emittierende Lichtquelle eine ad hoc Beobachtung des jeweils ausgesuchten Spektrums, so dass die Bestimmung der Messgröße des Analyten sehr schnell erfolgen kann. Allerdings sind die Intensitäten breitbandig emittierender Lichtquellen in der Regel gegenüber durchstimmbaren, bei einer konkreten Wellenlänge emittierenden Lichtquellen niedriger und zeigen eine geringere Güte.

Bevorzugt wird als Spektrum ein Reflexionsspektrum gewonnen. Gegenüber der Beobachtung eines Transmissionsspektrums ist man bei der Reflexionsspektroskopie nicht zwangsläufig auf dünne Körperstellen beschränkt, die überhaupt eine Beobachtung des transmittierten Lichts ermöglichen. Die Reflexionsspektroskopie bietet vielmehr die Möglichkeit, Körperstellen unabhängig von deren Dicke bis zu einer durch das beleuchtende Licht und dessen Intensität vorgegebenen Eindringtiefe zu untersuchen. Darüber hinaus kann bei einer Reflexionsspektroskopie auch der apparative Aufbau weniger komplex und insbesondere vergleichsweise raumsparend ausgeführt werden, da sich der Beleuchtungsstrahlengang und der Detektionsstrahlengang auf einer Seite des Körpers befinden.

In einer weiter bevorzugten Ausgestaltung werden zur Ermittlung der Mehrzahl an Werten für die Messgröße die Beobachtungsorte variiert, indem für mehrere Beobachtungsorte, die sich in ihrer Position in einer im Wesentlichen durch die Oberfläche des Köpers vorgegebenen X-Y-Ebene unterscheiden, jeweils weitere Beobachtungsorte, die sich in ihrer Position in einer im Wesentlichen durch die Tiefe des Körpers vorgegebenen Z-Richtung unterscheiden, gewählt werden. Der Körper wird gewissermaßen dreidimensional abgetastet, wobei an verschiedenen in der X-Y-Ebene festgelegten Beobachtungsorten jeweils ein Scan entlang der Z-Richtung in die Tiefe des Körpers hinein vorgenommen wird. Abhängig von der konkreten Aufgabenstellung genügt es insbesondere, in der X-Y-Ebene die Beobachtungsorte, für die jeweils ein Tiefenscan erfolgen soll, entlang einer eindimensionalen Trajektorie anzuordnen. Beispielsweise ist dies der Fall, wenn die Messgröße für einen Analyten in einem Gefäß, also beispielsweise in Blut, beobachtet werden soll. Mit einer eindimensionalen Trajektorie in der X-Y-Ebene können dabei leicht mehrere Gefäße überstrichen werden. Über den jeweiligen Tiefenscan bewegen sich die Beobachtungsorte dann in das Innere der entsprechend überstrichenen Blutgefäße hinein. Durch Festlegung einer vergleichsweise einfachen und insbesondere eindimensionalen Trajektorie in der X-Y-Ebene mit gegebenenfalls wenigen Beobachtungsorten kann durch wenige Tiefenscans rasch der gewünschte Beobachtungsort identifiziert und aus den dort gewonnenen Messdaten auf die Messgröße des untersuchten Analyten am Beobachtungsort geschlossen werden. Das Verfahren bietet insofern die Möglichkeit einer raschen und zuverlässigen Bestimmung der Messgröße des gewünschten Analyten, so dass es sich technisch in ein entsprechendes Produkt für den Consumer-Markt umsetzen lässt.

Vorteilhafterweise wird durch den Vergleich der ortsaufgelösten Werte der Messgröße auf die Lage eines Gefäßes oder eines Gewebes mit interstitiellem Wasser im Körper und auf den Wert der Messgröße des Analyten in einer Körperflüssigkeit im Gefäß oder im entsprechenden Gewebebereich geschlossen. Ein in einer Körperflüssigkeit enthaltener Analyt kommt in Blut, in Lymphe oder in interstitiellem Wasser des Körpers vor. In Fettgewebe, in Knochen oder Sehnen hingegen ist der entsprechende Analyt nicht enthalten. Durch eine Variation der Beobachtungsorte können somit mit den ortsaufgelöst enthaltenen Spektren im Körper Strukturen mit Analyt von Strukturen ohne Analyt voneinander unterschieden werden. Mit anderen Worten lassen sich insbesondere Fettgewebe, Knochen und Sehnen von Bereichen mit interstitiellem Wasser und Gefäßen unterscheiden. Bei entsprechender anatomischer Kenntnis kann insofern aus den ortsaufgelösten Spektren auf die konkrete anatomische Lage eines Gefäßes oder eines Gewebebereiches mit interstitiellem Wasser geschlossen, und hieraus die entsprechende Messgröße des Analyten bestimmt werden.

Vorteilhafterweise wird als Analyt Glucose betrachtet und als Messgröße der Glucose deren Konzentration bestimmt. Insbesondere kann das Verfahren insofern angewendet werden, um den Blutzuckerspiegel, also die Konzentration von Glucose im Blut, zu ermitteln. Zur jeweiligen Messung am Körper eignet sich insbesondere das Handgelenk, ein Unterarm oder ein Unterschenkel, da dort Blutgefäße einfach zugänglich sind. Durch einen Tiefenscan gelangt man über verschiedene Hautschichten ohne Glucose in Bereiche mit interstitiellem Wasser, also mit Glucose, durch Fettgewebe (ohne Glucose) und durch Gefäße. Abhängig von der Ortsauflösung wird man bei den Gefäßen weiter durch Gefäßwände und Muskelschichten bis in das blutführende Innere gelangen, wobei Glucose nicht ausschließlich im Blutstrom, sondern beispielsweise auch im Vasa Vasorum, in der Intima mit Endothel und in der Muskelschicht zu finden ist. Aus der konkreten Abfolge eines jeweiligen Tiefenscans kann jedoch infolge der bekannten Anatomie der Ort zur Bestimmung der entsprechenden Messgröße des Analyten festgelegt werden. Darüber hinaus erlaubt das angegebene Verfahren auch eine Unterscheidung zwischen Venen und Arterien, da sich diese voneinander durch die Dimensionen der bildenden Strukturen unterscheiden.

Zur Festlegung eines konkreten Wertes der Messgröße des Analyten wird in einer ersten Variante auf eine externe Kalibrierung zurückgegriffen. Beispielsweise kann dem Körper eine Körperflüssigkeit oder ein Körpergewebe mit einer bestimmten Konzentration eines Analyten entnommen und extern mit der für das angegebene Verfahren vorgesehenen Messapparatur untersucht werden. Bei der nicht-invasiven Messung unmittelbar am lebenden Körper wird dann ein Bezug zwischen dem erfassten Messsignal und dem aus der externen Messung bekannten Messsignal hergestellt und hieraus auf den konkreten Wert der im Körper erfassten Messgröße des Analyten geschlossen.

In einer bevorzugten zweiten Variante umfasst die Ermittlung des Wertes der Messgröße in einer bestimmten Struktur des Körpers eine innere bzw. intrinsische Kalibrierung durch Berücksichtigung wenigstens eines weiteren Wertes der Messgröße an einem anderen Beobachtungsort. Bei einem Analyten in Blut bieten hierzu gegebenenfalls die verschiedenen Konzentrationen in Arterie und Vene eine geeignete Möglichkeit zu einer Kalibrierung. Auch kann die Tatsache ausgenutzt werden, dass die Konzentrationen des Analyten in interstitiellem Wasser und in Blut zueinander korreliert sind. Auch kann der Patient zu einer inneren Kalibrierung insbesondere im Fall von Glucose den Analyten zu sich nehmen, und nachfolgend der zeitliche Verlauf des Anstiegs der Konzentration des Analyten im interstitiellen Wasser und in Blut beobachtet werden.

Die Variation der Beobachtungsorte erfolgt zweckmäßigerweise durch eine konfokale Spektroskopie. Um insbesondere unterschiedliche Strukturen in der Haut oder im Aufbau von Gefäßen unterscheiden zu können, wird das Verfahren zweckmäßigerweise mit einer Ortsauflösung im Sub-Millimeterbereich durchgeführt. Dabei erfolgt die Variation des Beobachtungsortes bevorzugt durch eine Variation der Abbildung einer detektorseitigen Blende in den Objektraum am Körper. Die Variation der Abbildung der Blende kann beispielsweise durch eine Verschiebung einer detektorseitig angeordneten Linse im Zusammenspiel mit der Blende erfolgen, so dass abhängig von der jeweiligen Verschiebung nur Licht eines konkreten Beobachtungsortes auf den Detektor gelangen kann. Die Auflösung und der Beobachtungsort sind somit insbesondere von der Größe bzw. der Lage des Bilds der Blende im Objektraum abhängig.

Bevorzugt wird insbesondere im Falle der konfokalen Spektroskopie Licht auf den Körper fokussiert, wobei die Fokuslage räumlich und zeitlich synchron zur Variation des Beobachtungsortes variiert wird. Die Beleuchtung geschieht dabei zweckmäßigerweise in einem zur Detektion parallelen Strahlengang. Durch die Fokussierung der Lichtquelle auf den jeweils ausgewählten Beobachtungsort wird die optische Energie, die mit der detektorseitigen Blende gesammelt werden kann, erhöht, wodurch sich im Detektor ein verbessertes Signal-zu-Rausch-Verhältnis ergibt.

Die zweitgenannte Aufgabe wird erfindungsgemäß gelöst durch eine Vorrichtung zur nicht-invasiven Bestimmung einer Messgröße eines Analyten in einem biologischen Körper, die einen Detektor zur spektralaufgelösten Erfassung eines vom Körper ausgehenden Lichts und eine Auswerteeinheit zur Auswertung und zur Analyse des vom Detektor erfassten Spektrums umfasst, wobei die Auswerteeinheit eingerichtet ist, in dem Spektrum einen für den Analyten charakteristischen Verlauf zu identifizieren und aus dem identifizierten Verlauf einen Wert für die Messgröße zu ermitteln. Des Weiteren umfasst die Vorrichtung eine steuerbare Optikeinheit, die ausgebildet ist, den Beobachtungsort am Körper zu variieren, und eine Steuereinheit, die eingerichtet ist, die Optikeinheit und die Auswerteeinheit derart anzusteuern, dass am Körper unter Variation des Beobachtungsortes eine Mehrzahl von Spektren gewonnen und hieraus eine Mehrzahl von jeweils eine Ortsinformation beinhaltenden Werten der Messgröße ermittelt wird, wobei die Auswerteeinheit zusätzlich dazu eingerichtet ist, durch einen Vergleich der ermittelten Werte der Messgröße eine Strukturinformation des Körpers abzuleiten und aus der Strukturinformation auf den Wert der Messgröße in einer bestimmten Struktur des Körpers zu schließen und diesen Wert der Messgröße auszugeben.

Die Steuereinheit und die Auswerteeinheit können gegebenenfalls in einer Einheit zusammengefasst sein. Die Optikeinheit kann insbesondere als ein verschiebbarer Optikkopf ausgebildet sein, der bei entsprechender Ansteuerung durch über eine Verschiebung insgesamt oder eines Teils seiner optischen Komponenten den Beobachtungsort am Körper festlegt.

Weitere vorteilhafte Ausgestaltungen ergeben sich aus den auf die Vorrichtung gerichteten Unteransprüchen. Dabei können die für das Verfahren jeweils genannten Vorteile sinngemäß auf die Vorrichtung übertragen werden.

Ausführungsbeispiele der Erfindung werden anhand einer Zeichnung näher erläutert. Dabei zeigen:
- Fig. 1: schematisch eine Vorrichtung zu einer optischen nicht-invasiven Bestimmung der Messgröße eines Analyten in einem Blutgefäß gemäß einer ersten Ausführungsvariante,
- Fig. 2: schematisch eine Vorrichtung zu einer optischen nicht-invasiven Bestimmung der Messgröße eines Analyten in einem Blutgefäß gemäß einer zweiten Ausführungsvariante, und
- Fig. 3: in einem Querschnitt schematisch eine Messanordnung mit einem einem Messkopf aufgelegten Handgelenk, wobei der Messkopf zur Bestimmung der Messgröße eines Analyten in einem Blutgefäß mittels Reflexionsspektroskopie ausgebildet ist.

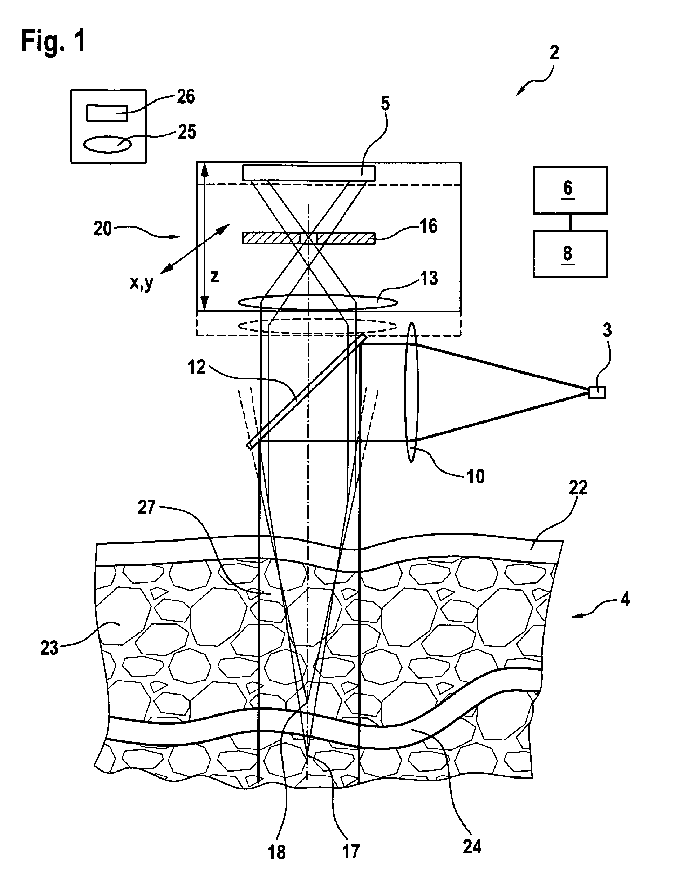

In Fig. 1 ist schematisch eine Vorrichtung 1 zur Bestimmung einer Messgröße eines Analyten in einem Blutgefäß mittels IR-Reflexionsspektroskopie dargestellt. Insbesondere ist die dargestellte Vorrichtung 1 zur Bestimmung der Konzentration von Glucose im Blut ausgebildet.

Die Vorrichtung 1 umfasst als eine Lichtquelle 3 zur Beleuchtung eines Körpers 4 mit Licht im IR-Spektralbereich einen durchstimmbaren Quantenkaskadenlaser. Der Quantenkaskadenlaser emittiert durchstimmbar Licht mit Wellenlängen zwischen 8,5 µm und 10,5 µm. Weiter ist ein Detektor 5 umfasst, der vom beleuchteten Körper 4 reflektiertes Licht erfasst. Eine Auswerteeinheit 6 bildet aus den erfassten Messsignalen des Detektors 5 ein Reflexionsspektrum und identifiziert hierin eine charakteristische Signatur des untersuchten Analyten. Insbesondere wird das Reflexionsspektrum hinsichtlich der Absorptionsbanden des Glucosemoleküls ausgewertet. Durch eine Steuereinheit 8 wird die Lichtquelle 3 durchgestimmt. Ebenfalls erfolgt über die Steuereinheit 8 eine Variation der Beobachtungsorte am Körper 4, was im Folgenden näher beschrieben wird.

Licht der Lichtquelle 3 wird über eine erste Linse 10 parallelisiert und über einen Strahlteiler 12 auf ein Beobachtungsvolumen 27 im Körper 4 gerichtet. Vom Beobachtungsvolumen 27 reflektiertes Licht durcheilt den Strahlteiler 12 und wird mittels einer zweiten Linse 13 auf eine Lochblende 16 fokussiert. Das durch die Lochblende 16 hindurchtretende Licht wird vom Detektor 5 erfasst.

Die zweite Linse 13 ist in Z-Richtung verschiebbar ausgestaltet. Durch eine Verschiebung in Z-Richtung wird das Bild der Lochblende 16 im Objektraum und damit der ausgewählte Beobachtungsort 17 entlang der Tiefe des Körpers 4 bewegt. Neben dem Beobachtungsort 17 ist ein bei entsprechender Verschiebung der zweiten Linse 13 gewanderter zweiter Beobachtungsort 18 eingezeichnet. Abhängig von der Position der zweiten Linse 13 kann nur Licht aus dem durch das Bild der Blende 16 im Objektraum definierten Beobachtungsort 17, 18 durch die Blende 16 auf den Detektor 5 gelangen.

Zusätzlich ist eine Verschiebung des Optikkopfes 20 in der X-Y-Ebene möglich, die im Wesentlichen durch die Oberfläche des Körpers 4 definiert ist. Dazu kann der Optikkopf 20 einen Teil der gezeigten optischen Komponenten umfassen, so dass das Bild der Blende 16 im Körper 4 entlang der X-Y-Ebene wandert. Im einfachsten Fall kann diese Bewegung des Beobachtungsortes ebenfalls durch eine Verschiebung der zweiten Linse 13 in der X-Y-Ebene erfolgen.

Zur Bestimmung der Messgröße des Analyten wird der Optikkopf 20 mittels der Steuereinheit 8 derart angesteuert, dass die Beobachtungsorte 17, 18 entlang einer vorgegebenen Bewegungstrajektorie in der X-Y-Ebene wandern, wobei für jede Position in der X-Y-Ebene ein Tiefenscan durch eine Verschiebung in Z-Richtung vorgenommen wird. Für jeden gewählten Beobachtungsort 17, 18 wird die Lichtquelle 3 im entsprechenden IR-Teilbereich durchgestimmt. Die Auswerteeinheit 6 erstellt für jeden Beobachtungsort 17, 18 aus den Messsignalen des Detektors 5 ein jeweiliges IR-Reflexionsspektrum. Aus einer vorgegebenen charakteristischen Signatur in den jeweiligen IR-Reflexionsspektren wird eine Mehrzahl von den Beobachtungsorten zugeordneten Werten der Messgröße des Analyten ermittelt.

Aus dem ortsaufgelösten Verlauf der ermittelten Werte der Messgröße des Analyten wird in der Auswerteeinheit 6 eine Strukturinformation über den anatomischen Aufbau des Körpers 4 erhalten. Beispielsweise kann abhängig von der erfassten Anwesenheit des Analyten auf Hautschichten 22, auf Fettgewebe 23 oder auf Blutgefäße 24 geschlossen werden. Durch die für jeden Beobachtungsort in der X-Y-Ebene vorgenommenen Tiefenscans und dem hieraus erfassten charakteristischen Verlauf der jeweiligen Werte der Messgröße des Analyten wird unter Zugrundelegen der bekannten Anatomie der oder diejenigen Beobachtungsorte festgelegt werden, die sich im Blutgefäß 24 befinden. Über eine entsprechende Kalibrierung wird dieser Wert für den konkreten Beobachtungsort innerhalb eines Blutgefäßes 24 über eine nicht dargestellte Ausgabeeinheit ausgegeben.

Zu einer Kalibrierung wird entweder Bezug genommen auf eine konkrete externe Messung. Alternativ wird aus dem Verlauf der ermittelten Werte der Messgröße des Analyten unter Hinzufügung der anatomischen Kenntnis eine innere Kalibrierung vorgenommen. Beispielsweise kann eine solche innere Kalibrierung vorgenommen werden, indem die für interstitielles Wasser erfassten Messwerte mit denjenigen Messwerten verglichen werden, die innerhalb eines Gefäßes 24 ermittelt sind. Abhängig von der Ortsauflösung der dargestellten Vorrichtung 1 können auch weitere Gefäßschichten zur Kalibrierung bzw. zur anatomischen Zuordnung der Beobachtungsorte 17, 18 herangezogen werden.

Sämtliche der vorgenannten Verfahrensschritte erfolgen vollautomatisiert ohne jedweden Eingriff durch ein Bedienpersonal oder dergleichen.

Zur Justierung der Vorrichtung 1 an oder auf dem Körper 4 ist weiter eine Abbildungslinse 25 und ein Kamerasystem 26 umfasst. Das Kamerasystem 26 beobachtet im sichtbaren Spektralbereich gegebenenfalls mit geringer Auflösung die Oberfläche des Körpers 4 und stellt das entsprechende Bild dem Patienten zur Verfügung. Durch eine bevorzugte Einblendung einer vorgegebenen Trajektorie in dieses Bild wird der Patient in die Lage gesetzt, die Vorrichtung 1 an die gewünschte und geeignete Stelle am Körper 4, beispielsweise an einem Handgelenk, am Unterarm oder am Unterschenkel, korrekt zu positionieren.

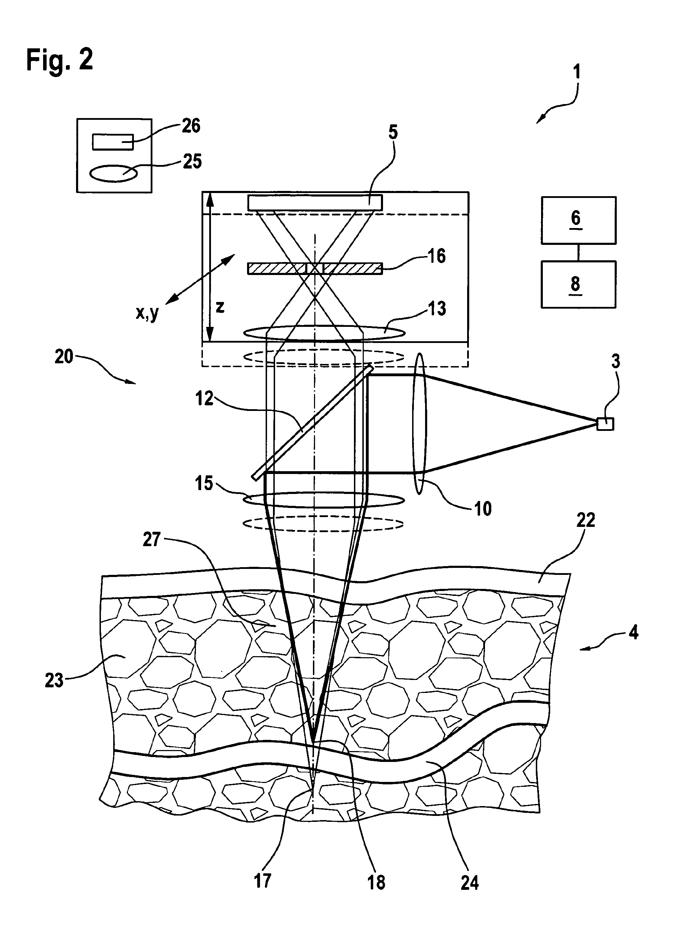

Die in Fig. 2 dargestellte alternative Variante einer Vorrichtung 2 zu einer optischen nicht-invasiven Erfassung der Messgröße eines Analyten in einem Blutgefäß mittels Reflexionsspektroskopie unterscheidet sich von der Vorrichtung 1 gemäß Fig. 1 dadurch, dass das Licht der Lichtquelle 3 mittels einer dritten Linse 15 in einem zur Detektion parallelen Strahlengang in das Beobachtungsvolumen 27 am Körper 4 fokussiert wird. Die Auswahl bzw. Variation der Beobachtungsorte 17, 18 erfolgt weiterhin durch ein Verschiebung der zweiten Linse 13 gegenüber der Lochblende 16. Zusätzlich wird räumlich und zeitlich synchron die dritte Linse 15 verschoben, so dass das Licht der Lichtquelle 3 stets auf den jeweils detektorseitig ausgewählten Beobachtungsort 17, 18 fokussiert ist. Hierdurch wird die Lichtausbeute auf dem Detektor 5 erhöht, wodurch sich das Signal-zu-Rausch Verhältnis verbessert wird.

Bei der Ausgestaltung entsprechend Fig. 1 erfolgt zur Auswahl des Beobachtungsortes 17, 18 eine Verschiebung optischer Elemente (vorliegend die zweite Linse 13) ausschließlich auf der Detektorseite, so dass sich ein kompakter Messkopf erstellen lässt. Durch die voluminöse Beleuchtung des Körpers 4 ist jedoch das Signal-zu-Rausch-Verhältnis nur sub-optimal. Bei der Variante gemäß Fig. 2 muss auf der Objektseite ein weiteres optisches Element (die dritte Linse 15) bewegt werden. Allerdings ist das Signal-zu-Rausch Verhältnis am Detektor 5 verbessert.

Beide Vorrichtungen 1, 2 gemäß Fig. 1 und 2 eignen sich insbesondere zu einer in-vivo nicht-invasiven Bestimmung des Blutzuckerspiegels, also der Konzentration von Glucose in Blut. Eine derartige Anwendung ist insofern interessant für die weltweit große Anzahl von an Diabetes erkrankten Patienten. Ohne eine gegebenenfalls schmerzhafte Entnahme von Blut kann mit den gezeigten Vorrichtungen 1, 2 leicht eine kontinuierliche und automatisierte Erfassung und Kontrolle des Blutzuckerspiegels zuhause geschehen.

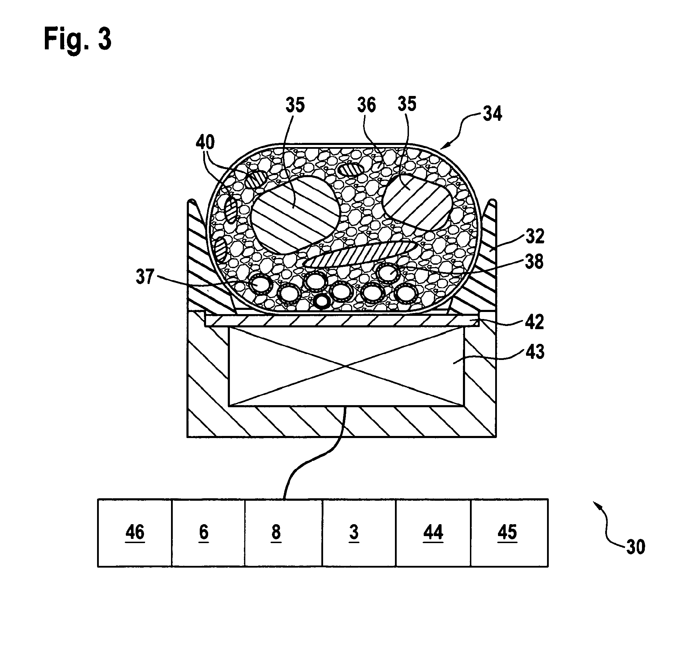

In Fig. 3 ist schematisch ein Messaufbau 30 zu einer eigenständigen Kontrolle des Blutzuckerspiegels entsprechend einem nicht-invasiven Verfahren dargestellt, wobei Gebrauch von den Vorrichtungen 1, 2 gemäß den Fig. 1 und 2 gemacht wird.

Auf einem Zentrierkissen 32 ist ein Arm 34 mit seinem Handgelenk positioniert. Im dargestellten Querschnitt sind Unterarmknochen 35, Gewebe 36, Arterien 37, Venen 38 und Muskeln bzw. Sehnen 40 erkennbar. Das Handgelenk des Arms 34 wird auf eine Kontaktplatte 32 aufgelegt. Hinter der Kontaktplatte ist ein Messkopf 43 positioniert, der entsprechend einer Vorrichtung 1 oder einer Vorrichtung 2 gemäß den Fig. 1 bzw. 2 ausgebildet sein kann. Im zum Messkopf 43 gehörenden eigentlichen Messgerät sind die Lichtquelle 3, die Auswerteeinheit 6 und die Steuereinheit 8 angeordnet. Daneben sind entsprechende Schnittstellen 44, ein Netzteil 45 und gegebenenfalls ein Monitor 46 vorgesehen. Auf dem Monitor 46 wird die ermittelte Konzentration von Glucose in Blut ausgegeben. Ebenfalls wird dort zur Positionierung des Arms 34 ein mit dem Kamerasystem 26 entsprechend Fig. 1 und 2 erfasstes Bild des Handgelenks angezeigt. Über eine eingeblendete Bewegungstrajektorie kann der Patient sein Handgelenk vor Beginn der Messung korrekt auf dem Messkopf 43 bzw. auf der Kontaktplatte 42 positionieren.

Zur Ausgabe des Messwerts kann im Fall einer gepulst betriebenen Lichtquelle 3 ein elektronisches Lock-In-Verfahren auf die gepulste Strahlung oder auf die Pulswiederholfrequenz angewendet werden, um die Qualität des Messwertes weiter zu verbessern.

Beispielhaft kann zur Ermittlung des Wertes der Messgröße, also insbesondere zur Ermittlung der Glucose-Konzentration, eine Kalibriermessung dadurch vorgenommen werden, dass nach einer insbesondere oralen Aufnahme des zu bestimmenden Analyten, eine Mehrzahl von Wiederholungsmessungen durchgeführt wird. Beispielsweise werden zeitverzögert um jeweils 30 Sekunden versetzt fünf Messungen durchgeführt und dabei jeweils an verschiedenen Messorten, wie in einer Vene, in einer Arterie und in einem Gewebe mit interstitiellem Wasser, der Anstieg der Konzentration des Analyten beobachtet. Aus dem jeweils beobachteten Anstieg werden eine zusätzliche Präzisierung und zumindest eine Validierung der Messwerte möglich.

### Bezugszeichenliste

- 1: Vorrichtung
- 2: Vorrichtung
- 3: Lichtquelle
- 4: Körper
- 5: Detektor
- 6: Auswerteeinheit
- 8: Steuereinheit
- 10: erste Linse
- 12: Strahlteiler
- 13: zweite Linse
- 15: dritte Linse
- 16: Lochblende
- 17: Beobachtungsort
- 18: Beobachtungsort
- 20: Optikeinheit
- 22: Hautschichten
- 23: Gewebe
- 24: Blutgefäß
- 25: Abbildungslinse
- 26: Kamerasystem
- 27: Beleuchtungsvolumen
- 30: Messaufbau
- 32: Zentrierkissen
- 34: Arm
- 35: Unterarmknochen
- 36: Gewebe
- 37: Arterie
- 38: Vene
- 40: Muskel, Sehne
- 42: Kontaktplatte
- 43: Messkopf
- 44: Schnittstelle
- 45: Netzteil
- 46: Monitor

## Patentansprüche

1. Verfahren zur nicht-invasiven Bestimmung einer Messgröße eines Analyten in einem biologischen Körper (4), wobei automatisiert von einem Körper (4) ausgehendes Licht unterschiedlicher Wellenlängen detektiert und ein Spektrum des detektierten Lichts gewonnen wird, und wobei in dem Spektrum ein für den Analyten charakteristischer Verlauf identifiziert und aus dem identifizierten Verlauf ein Wert für die Messgröße ermittelt wird,
**dadurch gekennzeichnet,**
**dass** am Körper (4) unter Variation des Beobachtungsortes (17, 18) eine Mehrzahl von Spektren gewonnen und hieraus eine Mehrzahl von jeweils eine Ortsinformation beinhaltenden Werten der Messgröße ermittelt wird, dass durch einen Vergleich der ermittelten Werte der Messgröße eine Strukturinformation des Körpers (4) abgeleitet und aus der Strukturinformation auf den Wert der Messgröße in einer bestimmten Struktur des Körpers (4) geschlossen wird, und dass dieser Wert der Messgröße ausgegeben wird.

2. Verfahren nach Anspruch 1,
**dadurch gekennzeichnet,**
**dass** der Körper (4) mit Licht unterschiedlicher Wellenlängen beleuchtet wird, und dass das vom beleuchteten Körper (4) ausgehende Licht detektiert wird, wobei bevorzugt vorgesehen ist,
**dass** der Körper (4) mit einer in einem IR-Teilbereich durchstimmbaren Lichtquelle (3), insbesondere mit einem Quantenkaskadenlaser, oder mit einer in einem IR-Teilbereich breitbandig emittierenden Lichtquelle (3), insbesondere mit einer Wärmequelle, beleuchtet wird.

3. Verfahren nach einem der vorhergehenden Ansprüche,
**dadurch gekennzeichnet,**
**dass** in dem Spektrum als ein charakteristischer Verlauf wenigstens ein Abschnitt einer spezifischen Spektralsignatur des Analyten identifiziert wird.

4. Verfahren nach einem der vorhergehenden Ansprüche,
**dadurch gekennzeichnet,**
**dass** als Spektrum ein Reflexionsspektrum gewonnen wird.

5. Verfahren nach einem der vorhergehenden Ansprüche,
**dadurch gekennzeichnet,**
**dass** zur Ermittlung der Mehrzahl an Werten für die Messgröße die Beobachtungsorte (17, 18) variiert werden, indem für mehrere Beobachtungsorte (17, 18), die sich in ihrer Position in einer im Wesentlichen durch die Oberfläche des Körpers (4) vorgegebenen X-Y-Ebene unterscheiden, jeweils weitere Beobachtungsorte (17, 18), die sich in ihrer Position in einer im Wesentlichen durch die Tiefe des Körpers (4) vorgegebenen Z-Richtung unterscheiden, gewählt werden.

6. Verfahren nach einem der vorhergehenden Ansprüche,
**dadurch gekennzeichnet,**
**dass** durch den Vergleich der Werte der Messgröße auf die Lage eines Gefäßes (24) im Körper (4) und auf den Wert der Messgröße des Analyten in einer Körperflüssigkeit im Gefäß (24) geschlossen wird.

7. Verfahren nach einem der vorhergehenden Ansprüche,
**dadurch gekennzeichnet,**
**dass** als Analyt Glucose betrachtet und als Messgröße der Glucose deren Konzentration bestimmt wird.

8. Verfahren nach einem der vorhergehenden Ansprüche,
**dadurch gekennzeichnet,**
**dass** die Ermittlung des Wertes der Messgröße in einer bestimmten Struktur des Körpers (4) eine innere Kalibrierung durch Berücksichtigung wenigstens eines weiteren Wertes der Messgröße an einem anderen Beobachtungsort (17, 18) umfasst.

9. Verfahren nach einem der vorgenannten Ansprüche,
**dadurch gekennzeichnet,**
**dass** die Variation des Beobachtungsortes (17, 18) durch eine Variation der Abbildung einer detektorseitigen Blende (16) in den Objektraum am Körper (4) erfolgt.

10. Verfahren nach einem der vorhergehenden Ansprüche,
**dadurch gekennzeichnet,**
**dass** Licht auf einen Beobachtungsort am Körper (4) fokussiert wird, wobei die Fokuslage räumlich und zeitlich synchron zur Variation des Beobachtungsortes (16) variiert wird.

11. Vorrichtung (1, 2) zur nicht-invasiven Bestimmung einer Messgröße eines Analyten in einem biologischen Körper (4), umfassend einen Detektor (5) zur Erfassung eines vom Körper (4) ausgehenden Lichts unterschiedlicher Wellenlängen, und eine Auswerteeinheit (6) zur Auswertung und zur Analyse des vom Detektor (5) erfassten Spektrums, die eingerichtet ist, in dem Spektrum einen für den Analyten charakteristischen Verlauf zu identifizieren und aus dem identifizierten Verlauf einen Wert für die Messgröße zu ermitteln,
**dadurch gekennzeichnet,**
**dass** weiter umfasst sind eine steuerbare Optikeinheit (20), die ausgebildet ist, den Beobachtungsort (17, 18) am Körper (4) zu variieren, und eine Steuereinheit (8), die eingerichtet ist, die Optikeinheit (20) und die Auswerteeinheit (6) derart anzusteuern, dass am Körper (4) unter Variation des Beobachtungsortes (17, 18) eine Mehrzahl von Spektren gewonnen und hieraus eine Mehrzahl von jeweils eine Ortsinformation beinhaltenden Werten der Messgröße ermittelt wird, und dass die Auswerteeinheit (6) zusätzlich dazu eingerichtet ist, durch einen Vergleich der ermittelten Werte der Messgröße eine Strukturinformation des Körpers (4) abzuleiten und aus der Strukturinformation auf den Wert der Messgröße in einer bestimmten Struktur des Körpers (4) zu schließen und diesen Wert der Messgröße auszugeben.

12. Vorrichtung (1, 2) nach Anspruch 11,
**dadurch gekennzeichnet,**
**dass** eine Lichtquelle (3) zur Beleuchtung des Körpers (4) mit Licht unterschiedlicher Wellenlängen umfasst ist, und dass der Detektor (5) zur Erfassung des vom beleuchteten Körper (4) ausgehenden Lichts eingerichtet und ausgebildet ist, wobei vorzugsweise weiterhin vorgesehen ist,
**dass** die Lichtquelle (3) in einem IR-Teilbereich durchstimmbar ist, insbesondere als ein Quantenkaskadenlaser gegeben ist, oder in einem IR-Teilbereich breitbandig emittiert, insbesondere als eine Wärmequelle gegeben ist.

13. Vorrichtung (1, 2) nach Anspruch 11 bis 12,
**dadurch gekennzeichnet,**
**dass** die Auswerteeinheit (6) eingerichtet ist, in dem Spektrum als einen charakteristischen Verlauf wenigstens einen Abschnitt einer spezifischen Spektralsignatur des Analyten zu identifizieren.

14. Vorrichtung (1, 2) nach einem der Ansprüche 11 bis 13,
**dadurch gekennzeichnet,**
**dass** die Detektoreinheit (5) zur Erfassung eines Reflexionsspektrums angeordnet und eingerichtet ist.

15. Vorrichtung (1, 2) nach einem der Ansprüche 11 bis 14,
**dadurch gekennzeichnet,**
**dass** die Steuereinheit (8) eingerichtet ist, die Optikeinheit (20) und die Auswerteeinheit (6) derart anzusteuern, dass zur Ermittlung der Mehrzahl an Werten für die Messgröße die Beobachtungsorte (17, 18) variiert werden, indem für mehrere Beobachtungsorte (17, 18), die sich in ihrer Position in einer im Wesentlichen durch die Oberfläche des Körpers (4) vorgegebenen XY-Ebene unterscheiden, jeweils weitere Beobachtungsorte (17, 18), die sich in ihrer Position in einer im Wesentlichen durch die Tiefe des Körpers (4) vorgegebenen Z-Richtung unterscheiden, gewählt werden.

16. Vorrichtung (1, 2) nach einem der Ansprüche 11 bis 15,
**dadurch gekennzeichnet,**
**dass** die Auswerteeinheit (6) eingerichtet ist, durch den Vergleich der Werte der Messgröße auf die Lage eines Gefäßes (24) im Körper (4) und auf den Wert der Messgröße des Analyten in einer Körperflüssigkeit im Gefäß (24) zu schließen.

17. Vorrichtung (1, 2) nach einem der Ansprüche 11 bis 16,
**dadurch gekennzeichnet,**
**dass** die Auswerteeinheit (6) eingerichtet ist, als Analyt Glucose zu betrachten und als Messgröße der Glucose deren Konzentration zu bestimmen.

18. Vorrichtung (1, 2) nach einem der Ansprüche 11 bis 17,
**dadurch gekennzeichnet,**
**dass** die Auswerteeinheit (6) eingerichtet ist, zur Ermittlung des Wertes der Messgröße in einer bestimmten Struktur des Körpers (4) eine innere Kalibrierung durch Berücksichtigung wenigstens eines weiteren Wertes der Messgröße an einem anderen Beobachtungsort (17, 18) vorzunehmen.

19. Vorrichtung (1, 2) nach einem der Ansprüche 11 bis 18,
**dadurch gekennzeichnet,**
**dass** im Detektionsstrahlengang eine Optikeinheit (20) angeordnet ist, die wenigstens eine Linse (15) und eine Blende (16) umfasst, wobei die Linse (15) und die Blende (16) relativ zueinander verschiebbar sind, und wobei die Steuereinheit (8) eingerichtet ist, zur Variation des Beobachtungsortes (17, 18) die Linse (15) und die Blende (16) relativ zueinander zu verschieben.

20. Vorrichtung (1, 2) nach einem der Ansprüche 11 bis 19,
**dadurch gekennzeichnet,**
**dass** im Beleuchtungsstrahlengang wenigstens eine verschiebbare Linse (13) umfasst ist, die das Licht der Lichtquelle (3) auf einen Beobachtungsort (17, 18) am Körper (4) fokussiert, wobei die Steuereinheit (8) eingerichtet ist, die Linse (13) räumlich und zeitlich synchron zur Variation des Beobachtungsortes (16) zu verschieben.

21. Vorrichtung (1, 2) nach einem der Ansprüche 11 bis 20,
**dadurch gekennzeichnet,**
**dass** zusätzlich ein Kamerasystem (26) zur Beobachtung und Anzeige des beleuchteten Körpers (4) vorgesehen ist.
